# EUROPEAN PATENT SPECIFICATION

(11) **EP 0 510 036 B1**
(45) Date of publication and mention of the grant of the patent: **19.04.1995**
(21) Application number: 91901788.9
(22) Date of filing: 09.01.1991
(51) Int. Cl.: C07D 403/12, C07D 401/12, A61K 31/41, A61K 31/415, A61K 31/44

(54) **2-SUBSTITUTED 4,5-DIPHENYL-IMIDAZOLES**
2-SUBSTITUIERTE 4,5-DIPHENYLIMIDAZOLE
4,5-DIPHENYL-IMIDAZOLES SUBSTITUES EN POSITION 2

(30) Priority: 12.01.1990 GB 9000696; 21.12.1990 GB 9027892
(43) Date of publication of application: 28.10.1992
(73) Proprietor: RHONE-POULENC RORER S.A., 92165 Antony Cédex (FR)
(72) Inventor: BRIDGE, Andrew, William, Dagenham Essex RM10 7XS (GB); HARRIS, Neil, Victor, Dagenham Essex RM10 7XS (GB); LYTHGOE, David, John, Dagenham Essex RM10 7XS (GB); SMITH, Christopher, Dagenham Essex RM10 7XS (GB)
(74) Representative: Bentham, Stephen
(86) International application number: EP9100023
(87) International publication number: WO9110662

(56) References cited:
- EP-A- 0 240 015
- EP-A- 0 359 197
- DE-A- 3 323 870
- Patent Abstracts of Japan, Vol 12, No 40, C474, abstract of JP 62-187469, publ 1988-02-05
- Chemical Abstracts, vol. 111, no. 11, 11 September 1989, (Columbus, Ohio, US), M. Tsuji et al: "Preparation of (heterocyclyalkylthio- or -sulfi- nyl)diphenylimidazoles as antiulcer and anti- inflammatory agents", abstract 97243c & JP 01 40,467 (HISAMITSU PHARMACEUTICAL CO INC.)

## Description

This invention in part relates to the use of therapeutically useful imidazole derivatives in the preparation pharmaceutical compositions for use in a method of treatment of the human or animal body. This invention also relates to therapeutically useful imidazole derivatives per se, to a process for their production and to pharmaceutical compositions containing them.

In Japanese Patent O.P.I. No. 64-40467 (Application No. 62-196117) there are disclosed inter alia compounds of the general formula (IA) hereinafter depicted, wherein XA represents hydrogen or halogen, or a lower alkyl or lower alkoxy group, m represents 0 or 1, n represents 1, 2, 3 or 4, and RA represents a heterocyclic ring, for example pyridyl, pyrimidinyl, thiazinyl, thiazolyl, imidazolinyl, imidazolidinyl, and their acid addition salts, alone and in association with pharmaceutically acceptable carriers and coatings, and a process for their preparation. The specification states that lower alkyl can have 1 to 6 carbon atoms such as methyl, ethyl, propyl or butyl, and lower alkoxy can be methoxy, ethoxy, n-propoxy, iso-propoxy, n-butoxy, iso-butoxy, or tert-butoxy etc. containing up to 6 carbon atoms, and the said heterocyclic ring can be pyridyl, pyrimidinyl or imidazolinyl, substituted or unsubstituted with less than 3 substituents, the substituents including methyl, ethyl, n-propyl, iso-propyl, n-butyl n-pentyl or iso-pentyl etc. with 1 to 6 carbon atoms, halogen or lower alkoxy.

However, in that specification, those compounds are described as having anti-ulcer and anti-inflammatory activity. Nowhere in that specification is there any suggestion that the compounds and their pharmaceutical compositions could be useful as inhibitors of acyl coenzyme-A:cholesterol-O-acyl transferase or in the treatment of conditions such as atherosclerosis, hyperlipidaemia, cholesterol ester storage disease and atheroma in vein grafts, or as inhibitors of the binding of thromboxane TxA₂ to its receptors or in the treatment of conditions such as thrombosis and myocardial infarction, vasospastic disorders and bronchospasm or in reperfusion salvage therapy.

In the specification of European Patent Application Publication No. 0301422 A2 there are disclosed inter alia compounds of the general formula:-

Het-S(O)_{nb}-CH₂-A (IB)

hereinafter depicted, wherein Het is a 4,5-diphenylimidazol-2-yl group , nb is 0 or 1, and A is a group of general formula (IIB) hereinafter depicted, wherein R_{4'}, R_{4''} and R_{4'''} are each hydrogen or C₁₋₄ alkyl, and physiologically acceptable salts thereof, alone and in association with pharmaceutically acceptable carriers and coatings, and a process for their preparation.

However, in that specification, those compounds are described as having anti-ulcer and gastric acid antisecretory activities. Nowhere in that specification is there any suggestion that the compounds and their pharmaceutical compositions could be useful as inhibitors of acyl coenzyme-A:cholesterol-O-acyl transferase or in the treatment of conditions such as atherosclerosis, hyperlipidaemia, cholesterol ester storage disease and atheroma in vein grafts, or as inhibitors of the binding of thromboxane TxA₂ to its receptors or in the treatment of conditions such as thrombosis and myocardial infarction, vasospastic disorders and bronchospasm or in reperfusion salvage JP-A-62-187469 discloses, inter alia, 4,5-diphenyl-2-(2-pyridylmethyl)thioimidazole and its sulfinyl analogue as useful for treating thrombosis and atherosclerosis. There is, however, no disclosure of the inhibition of the acyl coenzyme-A:cholesterol-O-acyl transferase described above.

Thus, the utilities disclosed hereinafter in the present specification are entirely unexpected.

Accordingly, the present invention provides the use of a compound of the general formula:-

[DPIM]-S(O)ₚ-W-Y (I)

wherein [DPIM] is as hereinafter depicted, wherein R and R¹ are the same or different and each represents a hydrogen or halogen (i.e. fluorine, chlorine, bromine or iodine) atom or a straight- or branched-chain alkyl or alkoxy group containing from 1 to about 6, preferably from 1 to 3 carbon atoms, p represents 0, 1 or 2, W represents a methylene group or an alkylene chain of 2 to about 6 carbon atoms optionally substituted with one or more straight- or branched-chain alkyl groups of 1 to about 4 carbon atoms, Y represents a 5- or 6-membered unsaturated ring containing 1, 2, 3 or 4 nitrogen atoms, optionally substituted by one or more substituents selected from straight- and branched-chain alkyl groups containing from 1 to about 6 carbon atoms (which are each optionally substituted by a phenyl group), phenyl groups, amino groups and nitro groups, and pharmaceutically acceptable acid addition salts thereof, provided that when p represents 0 or 1 and W represents an unsubstituted methylene group, Y represents other than a 2-pyridyl group, for the preparation of a pharmaceutical composition for the treatment of conditions which can be ameliorated by the administration of an inhibitor of acyl coenzyme-A:cholesterol-O-acyl transferase (ACAT; EC 2.3.1.26), such as atherosclerosis, hyperlipidaemia, cholesterol ester storage disease and atheroma in vein grafts, or of an inhibitor of the binding of thromboxane TxA₂ to its receptors, such as thrombosis and myocardial infarction, vasospastic disorders, for example associated with angina, and bronchospasm, for example associated with asthma, or in reperfusion salvage therapy, for example after ischaemic injury.

The invention also provides a compound of formula (I), or a pharmaceutically acceptable acid addition salt thereof, as hereinbefore defined, subject to the provisos that (i) when p represents 0 or 1 and W represents an unsubstituted methylene group or an unsubstituted alkylene chain of 2 to 4 carbon atoms, Y represents other than a pyridyl, pyrimidinyl or imidazolinyl group, substituted or unsubstituted with 1 or 2 alkyl groups, R and R¹ being as hereinbefore defined, and (ii) when R and R¹ each represent hydrogen atoms, p represents 0 or 1 and W represents an unsubstituted methylene group, Y represents other than a pyrid-2-yl group, substituted or unsubstituted with 1, 2 or 3 alkyl groups each containing up to 4 carbon atoms, for use in a new method of treatment, of the human or animal body, by therapy, of conditions which can be ameliorated by the administration of an inhibitor of acyl coenzyme-A:cholesterol-O-acyl transferase, such as atherosclerosis, hyperlipidaemia, cholesterol ester storage disease and atheroma in vein grafts, or of an inhibitor of the binding of thromboxane TxA₂ to its receptors, such as thrombosis and myocardial infarction, vasospastic disorders, for example associated with angina, and bronchospasm, for example associated with asthma, or in reperfusion salvage therapy, for example after ischaemic injury.

The present invention also provides pharmaceutical formulations which contain at least one of the compounds of formula (I), or a pharmaceutically acceptable salt thereof, as hereinbefore defined, subject to the provisos that (i) when p represents 0 or 1 and W represents an unsubstituted methylene group or an unsubstituted alkylene chain of 2 to 4 carbon atoms, Y represents other than a pyridyl, pyrimidinyl or imidazolinyl group, substituted or unsubstituted with 1 or 2 alkyl groups, R and R¹ being as hereinbefore defined, and (ii) when R and R¹ each represent hydrogen atoms, p represents 0 or 1 and W represents an unsubstituted methylene group, Y represents other than a pyrid-2-yl group, substituted or unsubstituted with 1, 2 or 3 alkyl groups each containing up to 4 carbon atoms, in association with a pharmaceutically acceptable carrier or coating.

The present invention also provides new compounds of formula (I), and pharmaceutically acceptable salts thereof, as hereinbefore defined, subject to the provisos that (i) when p represents 0 or 1 and W represents an unsubstituted methylene group or an unsubstituted alkylene chain of 2 to 4 carbon atoms, Y represents other than a pyridyl, pyrimidinyl or imidazolinyl group, substituted or unsubstituted with 1 or 2 alkyl groups, R and R¹ being as hereinbefore defined, and (ii) when R and R¹ each represent hydrogen atoms, p represents 0 or 1 and W represents an unsubstituted methylene group, Y represents other than a pyrid-2-yl group, substituted or unsubstituted with 1, 2 or 3 alkyl groups each containing up to 4 carbon atoms.

Especially important features of the present invention are, or involve, compounds of general formula (I) wherein at least one of the symbols has a value selected from the following:-
(i) R represents a hydrogen or fluorine atom or a methyl or methoxy group;
(ii) R¹ represents a hydrogen or fluorine atom or a methyl or methoxy group;
(iii) W represents a methylene or ethylidene group or an alkylene chain of 2 to 6 carbon atoms;
(iv) Y represents an imidazolyl (preferably imidazol-1-yl or imidazol-2-yl), pyrazolyl (preferably pyrazol-1-yl), triazolyl (preferably 1,2,4-triazol-1-yl), tetrazolyl (preferably tetrazol-1-yl or tetrazol―2-yl), pyridyl (preferably pyrid-4-yl) or pyrrolyl (preferably pyrrol-1-yl) group, optionally substituted by one or two substituents selected from methyl, ethyl, benzyl, phenyl, amino and nitro groups; the other symbols being as hereinbefore defined, and pharmaceutically acceptable acid addition salts thereof.

Preferred acid addition salts are the hydrochlorides and maleates.

Important compounds according to the invention include:
- 1A: 2-[3-(2-methylimidazol-1-yl)propylthio]-4,5-diphenylimidazole and its dihydrochloride
- 1B: 2-[3-(pyrazol-1-yl)propylthio]-4,5-diphenylimidazole and its hydrochloride
- 1C: 2-[2-(1,2,4-triazol-1-yl)ethylthio]-4,5-diphenylimidazole
- 1D: 2-[3-(1,2,4-triazol-1-yl)propylthio]-4,5-diphenylimidazole
- 1E: 2-[4-(1,2,4-triazol-1-yl)butylthio]-4,5-diphenylimidazole
- 1F: 2-[5-(1,2,4-triazol-1-yl)pentylthio]-4,5-diphenylimidazole and its dihydrochloride
- 1G: 2-[2-(imidazol-1-yl)ethylthiol-4,5-diphenylimidazole
- 1H: 2-[3-(imidazol-1-yl)propylthio]-4,5-diphenylimidazole
- 1I: (±)-2-[1-(imidazol-1-yl)ethylthio]-4,5-diphenylimidazole and its dihydrochloride
- 1J: 2-[(1-methylimidazol-2-yl)methylthio]-4,5-diphenylimidazole
- 1JA: 2-[(1-methylimidazol-2-yl)methylthio]-4,5-diphenylimidazole hydrochloride
- 1K: 2-[(1-benzylimidazol-2-yl)methylthio]-4,5-diphenylimidazole
- 1L: 2-[6-(1,2,4-triazol-1-yl)hexylthio]-4,5-diphenylimidazole
- 1M: 2-[4-(2-methylimidazol-1-yl)butylthio]-4,5-diphenylimidazole
- 1N: 2-[3-(2-phenylimidazol-1-yl)propylthio]-4,5-diphenylimidazole
- 1O: 2-[2-(2-methyl-5-nitroimidazol-1-yl)ethylthio]-4,5-diphenylimidazole
- 1P: 2-[3-(3,5-dimethylpyrazol-1-yl)propylthio]-4,5-diphenylimidazole
- 1Q: 2-[3-(tetrazol-1-yl)propylthio]-4,5-diphenylimidazole
- 1R: 2-[3-(tetrazol-2-yl)propylthio]-4,5-diphenylimidazole
- 1S: 2-[4-(tetrazol-1-yl)butylthio]-4,5-diphenylimidazole
- 1T: 2-[4-(tetrazol-2-yl)butylthio]-4,5-diphenylimidazole
- 1U: 2-[pyrid-4-ylmethylthio]-4,5-diphenylimidazole and its dihydrochloride
- 2A: 2-[5-(3,5-dimethylpyrazol-1-yl)pentylthio]-4,5-diphenylimidazole
- 2AA: 2-[5-(3,5-dimethylpyrazol-1-yl)pentylthio]-4,5-diphenylimidazole dihydrochloride
- 2B: 2-[5-(2-ethylimidazol-1-yl)pentylthio]-4,5-diphenylimidazole
- 2C: 2-[5-(imidazol-1-yl)pentylthio]-4,5-diphenylimidazole,
- 2D: 2-[5-(2-methylimidazol-1-yl)pentylthio]-4,5-diphenylimidazole
- 2E: 2-[6-(2-methylimidazol-1-yl)hexylthio]-4,5-diphenylimidazole
- 2F: 2-[6-(2-ethylimidazol-1-yl)hexylthio]-4,5-diphenylimidazole
- 2G: 2-[5-(pyrazol-1-yl)pentylthio]-4,5-diphenylimidazole
- 2H: 2-[6-(imidazol-1-yl)hexylthio]-4,5-diphenylimidazole
- 2I: 2-[6-(pyrazol-1-yl)hexylthio]-4,5-diphenylimidazole
- 2J: 2-[5-(3-methylpyrazol-1-yl)pentylthio]-4,5-diphenylimidazole and 2-[5-(5-methylpyrazol-1-yl)pentylthio]-4,5-diphenylimidazole
- 2K: 2-[5-(3-amino-5-methylpyrazol-1-yl)pentylthio]-4,5-diphenylimidazole
- 2L: 2-[5-(4-bromo-3,5-dimethylpyrazol-1-yl)pentylthio]-4,5-diphenylimidazole
- 2M: 2-[5-(3,5-diphenylpyrazol-1-yl)pentylthio]-4,5-diphenylimidazole
- 2N: 2-[5-(pyrrol-1-yl)pentylthio]-4,5-diphenylimidazole
- 2O: 2-[5-(2,5-dimethylpyrrol-1-yl)pentylthio]-4,5-diphenylimidazole
- 2P: 2-[4-(3,5-dimethylpyrazol-1-yl)butylthio]-4,5-diphenylimidazole
- 2Q: 2-[6-(3,5-dimethylpyrazol-1-yl)hexylthio]-4,5-diphenylimidazole
- 2R: 2-[5-(3,5-dimethylpyrazol-1-yl)pentylthio]-4,5-(bis-4-fluorophenyl)imidazole
- 2RA: 2-[5-(3,5-dimethylpyrazol-1-yl)pentylthio]-4,5-(bis-4-fluorophenyl)imidazole hydrochloride
- 2S: 2-[5-(3,5-dimethylpyrazol-1-yl)pentylthio]-4,5-(bis-4-methylphenyl)imidazole
- 2SA: 2-[5-(3,5-dimethylpyrazol-1-yl)pentylthio]-4,5-(bis-4-methylphenyl)imidazole hydrochloride
- 2T: 2-[5-(3,5-dimethylpyrazol-1-yl)pentylthio]-4,5-(bis-4-methoxyphenyl)imidazole
- 2U: 2-(4,5-diphenylimidazol-2-ylthiomethyl)imidazole
- 2UA: 2-(4,5-diphenylimidazol-2-ylthiomethyl)imidazole dimaleate
- 2V: 4,5-diphenyl-2-[3-(1-methylimidazol-2-yl)propylthio]imidazole
- 2W: 2-[(5-(3,5-dimethylpyrazol-1-yl)pent-1-yl)sulphonyl]-4,5-diphenylimidazole and
- 2X: 2-[(5-(3,5-dimethylpyrazol-1-yl)pent-1-yl)sulphinyl]-4,5-diphenylimidazole.

The letters 1A to 2X are allocated to compounds for easy reference later in this specification.

The compounds according to the invention are inhibitors of acyl coenzyme-A:cholesterol-O-acyl transferase (ACAT; EC 2.3.1.26). They are therefore of value as anti-atherosclerotic agents and have utility in the treatment of conditions such as atherosclerosis, hyperlipidaemia, cholesterol ester storage disease and atheroma in vein grafts.

They are also inhibitors of the binding of thromboxane TxA₂ to its receptors. They are therefore of utility in the treatment of conditions such as thrombosis and myocardial infarction, vasospastic disorders, for example associated with angina, and bronchospasm, for example associated with asthma, or in reperfusion salvage therapy, for example after ischaemic injury.

Compounds within the scope of the present invention exhibit positive pharmacological activities as demonstrated by the following in-vitro and in-vivo tests which are believed to correlate to pharmacological activity in humans and other animals.

In in-vitro tests on human platelet membrane, compounds of the invention produced up to 50% inhibition of the binding of thromboxane TxA₂ to its receptors at concentrations down to about 600 nanomolar or less.

In assays performed in-vitro, microsomes, obtained from the livers of rats fed on a diet supplemented with 0.5%w/w cholesterol and 0.25%w/w cholic acid for 7 days, were incubated with radiolabelled oleoyl-CoA in the presence of compounds according to the invention at a concentration of 0.5 or 1 »g/ml. The degree of ACAT inhibition produced is shown in Table 1.

In in-vivo tests, using rats fed on a similar diet to that above and further supplemented by 0.03% w/w of test compound, the compounds according to the invention inhibited increases in plasma cholesterol concentrations, measured after 3 days, relative to control animals fed on the cholesterol supplemented diet without the drug, as shown in Table 1.

**Table 1**

| COMPOUND | In-vitro % Inhibition at | | In-vivo % Inhibition |
|---|---|---|---|
| | 1»g/ml | 0.5»g/ml | |
| 1A | 90 | | 64 |
| 1B | 83 | | |
| 1C | 88 | | |
| 1D | 84 | | |
| 1E | 86 | | 53 |
| 1F | 92 | | 74 |
| 1G | 86 | | |
| 1H | 80 | | |
| 1I | 53 | | |
| 1J | 85 | | |
| 1JA | 82 | | 23 |
| 1K | 57 | | |
| 1L | 91 | | |
| 1M | | 87 | 26 |
| 1N | | 57 | |
| 1O | | 82 | |
| 1P | | 87 | |
| 1Q | 88 | | |
| 1R | 80 | | |
| 1S | | 84 | 41 |
| 1T | | 74 | |
| 1U | 71 | | |

Compounds of general formula (I) can be prepared by the application or adaptation of known methods, by which is meant methods used heretofore or described in the literature.

According to a feature of the invention, the new compounds of general formula (I), as hereinbefore defined, are prepared by the reaction of a compound of the general formula:-

[DPIM]-SH (II)

or a salt thereof, wherein [DPIM] is as hereinbefore defined, with a compound of formula:

X-W-Y (III)

or a salt thereof, wherein X is a group displaceable by a thiolate salt, such as a halogen e.g. a chlorine, bromine or iodine, atom or an alkyl- or aryl-sulphonyloxy group (e.g. methanesulphonyloxy or 4-toluenesulphonyloxy) and W and Y are subject to the same provisos as hereinbefore defined. The reaction is generally carried out in an inert organic solvent such as tetrahydrofuran or dimethylformamide at a temperature from ambient to 110°C and optionally in the presence of a proton acceptor, such as an amine (e.g. triethylamine).

According to a further feature of the present invention, the new compounds of general formula (I) are prepared by the interconversion of other compounds of general formula (I). For example, compounds of formula (I), wherein [DPIM], R, R¹, W and Y are subject to the same provisos as hereinbefore defined and p represents 1 or 2, are prepared by the oxidation of compounds of formula (I), wherein [DPIM], R, R¹, W and Y are as hereinbefore defined and p is less than in the desired product.

The oxidation may be performed by using a conventional oxidant, such as hydrogen peroxide, sodium metaperiodate, a hypochlorite, an acyl nitrite, sodium perborate, peracids, such as percarboxylic acids (e.g. m-chloroperbenzoic acid), potassium permanganate or potassium hydrogen persulphate, or a ruthenium (VIII) compound, in an inert solvent, at or below room temperature. Ruthenium (VIII) compounds can conveniently be prepared in situ by the oxidation of ruthenium (III) compounds, for example by means of a percarboxylic acid (e.g. m-chloroperbenzoic acid).

Suitable solvents may include water, alcohols, water-alcohol mixtures, chlorinated hydrocarbons, such as dichloromethane, and organic acids.

Compounds of general formula (I) wherein p is 1, the other symbols being as hereinbefore defined, may be obtained in a chirally pure form by separation of the enantiomers arising from a non-selective oxidation or by using known enantio-selective oxidising systems.

By the term "pharmaceutically acceptable salts" as used in this specification is meant salts the anions of which are relatively innocuous to the animal organism when used in therapeutic doses so that the beneficial pharmaceutical properties of the parent compounds of general formula (I) are not vitiated by side-effects ascribable to those anions.

Suitable acid addition salts for use in pharmaceuticals may be selected from salts derived from inorganic acids, for example hydrochlorides, hydrobromides, phosphates, sulphates and nitrates, and organic acids, for example oxalates, lactates, tartrates, acetates, salicylates, citrates, propionates, succinates, fumarates, maleates, methylene-bis-β-hydroxynaphthoates, gentisates and di-p-toluoyltartrates.

According to a further feature of the invention, salts of compounds of formula (I) are prepared by reaction of the parent compounds of formula (I) with the appropriate acid, by the application or adaptation of known methods.

As well as being useful in themselves as active compounds, salts of compounds of formula (I) are useful for the purposes of purification of the parent compounds of formula (I), for example by exploitation of the solubility differences between the salts and the parent compounds, by tcchniques well known to those skilled in the art.

The parent compounds of formula (I) can be regenerated from their salts by the application or adaptation of known methods.

For example, parent compounds of general formula (I) can be regenerated from their acid addition salts by treatment with an alkali, e.g. aqueous sodium bicarbonate solution or aqueous ammonia solution.

In this specification reference to compounds of formula (I) is intended to include reference to their pharmaceutically acceptable salts, where the context so permits.

The starting materials and intermediates can be prepared by the application or adaptation of known methods.

Compounds of formula (I) can be purified by the usual physical means, for example by crystallisation or chromatography.

The following Examples illustrate the preparation of the compounds according to the invention.

N.M.R. spectra were recorded at 200MHz or 400MHz. Chemical shifts are expressed in ppm relative to tetramethylsilane. Abbreviations have the following significances:-
s = singlet, d = doublet, t = triplet, q = quartet, m = multiplet, dd = doublet of doublets.

Infra-red spectra were recorded in potassium bromide discs. The positions of the major absorption peaks are given.

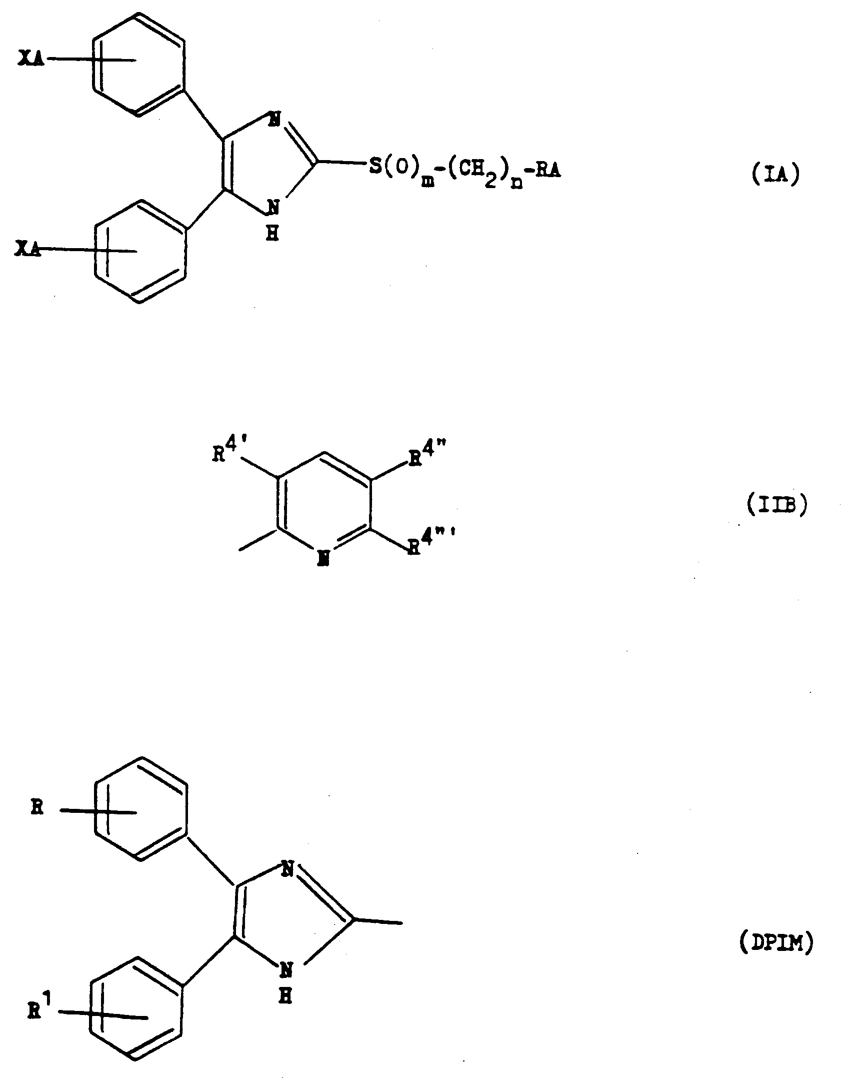

### EXAMPLE 1

### Compounds 1A to 1H and 1L to 1P

i) Sodium hydride (80% dispersion in oil, 1.65g, 50mmol) was added to a stirred solution of 2-methylimidazole (4.1g, 50mmol) in anhydrous dimethylformamide (50ml) with ice cooling. After stirring for 30min 1-bromo-3-chloro-propane (7.9g, 50mmol) was added and the mixture allowed to stir at room temperature overnight. Evaporation of the mixture gave a colourless semisolid which was partitioned between ether (150ml) and water (100ml). The layers were separated and the ether layer washed with water (3x50ml), dried (MgSO₄) and evaporated to give 1-(3-chloropropyl)-2-methylimidazole (2.3g), as a colourless oil.

Potassium t-butoxide (1.56g, 14mmol) was added to a stirred solution of 4,5-diphenylimidazole-2-thiol (3.5g, 14mmol) in anhydrous dimethylformamide (50ml). After stirring at room temperature for 30min, 1-(3-chloro-propyl)-2-methylimidazole (2.3g, 15mmol) was added and the mixture stirred at room temperature overnight. The mixture was evaporated to dryness and the residue partitioned between ethyl acetate (150ml) and water (50ml). The layers were separated and the organic layer was washed with water (50m), dried (MgSO₄) and evaporated. The resulting semisolid was purified by flash chromatography (10% ethanol in ethyl acetate) to give 2-[3-(2-methylimidazol-1-yl)propylthio]-4,5-diphenylimidazole as a white solid.

A solution of the solid in ethanol (30ml) was added to a stirred solution of hydrogen chloride in ethanol [prepared by adding acetyl chloride (20ml) dropwise to cold (10°C) ethanol (90ml), with stirring, maintaining the temperature below 30°C] and the mixture stirred at room temperature for 2hr. Evaporation gave a white solid, which was triturated with ether and collected by filtration to give 2-[3-(2-methylimidazol-1-yl)propylthio]-4,5-diphenylimidazole dlhydrochloride (1.9g), as a white solid, m.p. 252-253°C;
[Found:- C, 59.0; H, 5.4; N, 12.6; Cl, 15.6; S, 7.3%
Calculated for C₂₂H₂₂N₄S.2HCl:- C, 59.1; H, 5.4; N, 12.5; Cl, 15.9; S, 7.2%
N.M.R. (200MHz; CD₃SOCD₃): 2.19 (2H, m), 2.67 (3H, s), 3.49 (2H, t, J=7Hz), 4.29 (2H, t, J=6Hz), 7.40-7.60 (11H, m), 7.78 (1H, d, J=2Hz)
IR (KBr): 775, 1506, 2510, 2643, 3432 cm⁻¹].

By proceeding in a similar manner, but replacing the 2-methylimidazole by the appropriate azole and the 1-bromo-3-chloropropane by the appropriate dihalide, and with the salt formation step being optional, there were prepared:-
ii) 2-[3-(pyrazol-1-yl)propylthio]-4,5-diphenylimidazole hydrochloride, m.p. 174-175°C;
iii) 2-[2-(1,2,4-triazol-1-yl)ethylthio]-4,5-diphenylimidazole, m.p. 144-145°C;
iv) 2-[3-(1,2,4-triazol-1-yl)propylthio]-4,5-diphenylimidazole, m.p. 127-129°C;
v) 2-[4-(1,2,4-triazol-1-yl)butylthio]-4,5-diphenylimidazole, m.p. 117-118°C;
vi) 2-[5-(1,2,4-triazol-1-yl)pentylthio]-4,5-diphenylimidazole dihydrochloride, m.p. 253-254°C;
vii) 2-[2-(imidazol-1-yl)ethylthio]-4,5-diphenylimidazole, m.p. 172-173°C;
viii) 2-[3-(imidazol-1-yl)propylthio]-4,5-diphenylimidazole, m.p. 184°C;
ix) 2-[6-(1,2,4-triazol-1-yl)hexylthio]-4,5-diphenylimidazole, m.p. 69-70°C;
x) 2-[4-(2-methylimidazol-1-yl)butylthio]-4,5-diphenylimidazole, m.p. 168-169°C;
xi) 2-[3-(2-phenylimidazol-1-yl)propylthio]-4,5-diphenylimidazole, m.p. 142-145°C;
xii) 2-[2-(2-methyl-5-nitroimidazol-1-yl)ethylthio]-4,5-diphenylimidazole, m.p. 217-220°C; and
xiii) 2-[3-(3,5-dimethylpyrazol-1-yl)propylthio]-4,5-diphenylimidazole, m.p. 119-120°C.

### EXAMPLE 2

### Compound 1I

Potassium t-butoxide (7.84g, 70mmol) was added to a stirred solution of 4,5-diphenylimidazole-2-thiol (5.0g, 20mmol) in anhydrous dimethylformamide (100ml). After stirring at room temperature for 30min, (±)-1-(1-chloroethyl)imidazole hydrochloride (7.3g, 44mmol) was added and the mixture stirred at room temperature overnight. Work up and purification by flash chromatography (25% methanol in ethyl acetate) as in Example 1(i) gave the free base as an orange oil. This was converted to the hydrochloride as in Example 1(i) to give (±)-2-[1-(imidazol-1-yl)ethylthio]-4,5-diphenylimidazole dihydrochloride (2.5g), as an off-white powder, m.p. 169-170°C;
[Found:- C, 55.2; H, 5.0; N, 12.5; Cl, 15.7; S, 7.4%
Calculated for C₂₀H₁₈N₄S.2HCl.H₂O:- C, 54.9; H, 5.0; N, 12.8; Cl, 16.2; S, 7.3%
N.M.R. (200MHz; CD₃SOCD₃): 2.04 (3H, d, J=8Hz), 6.53 (1H, q, J=8Hz), 7.40-7.55 (10H, m), 7.80 (1H, d, J=2Hz), 8.07 (1H, d, J=2Hz), 9.49 (1H, s,)
IR (KBr): 696, 766, 2637, 2697, 2713, 2800, 2828 cm⁻¹].

### EXAMPLE 3

### Compounds 1J, 1JA, 1K and 1U

i) Sodium hydride (80% dispersion in oil, 3.27g, 100mmol) was added to a stirred suspension of 4,5-diphenylimidazole-2-thiol (13.8g, 54mmol) in anhydrous tetrahydrofuran (150ml). The mixture was stirred at 60°C until complete solution occurred. 1-Methyl-2-chloromethylimidazole hydrochloride (10.0g, 60mmol) was added and the mixture stirred at reflux overnight. After cooling to room temperature the mixture was poured into water (500ml) and the cream precipitate collected by filtration. Recrystallisation from isopropanol gave 2-[(1-methylimidazol-2-yl)methylthio]-4,5-diphenylimidazole (12.2g), as a yellow powder, m.p. 189-190°C; [Found:- C, 69.2; H, 5.3; N, 16.3; S, 9.1%; Calculated for C₂₀H₁₈N₄S:- C, 69.3; H, 5.2; N, 16.2; S, 9.3.%
   N.M.R. (400MHz; CDCl₃): 3.68 (3H, s), 4.24 (2H, s), 6.94 (1H, d, J=2Hz), 7.01 (1H, d, J=2Hz), 7.20-7.60 (10H, m,)
   IR (KBr): 689, 764, 1439, 1644, 3406 cm⁻¹].
ii) The free base was converted to the hydrochloride salt as in Example 1(i), m.p. 216-221°C;
   [Found:- C, 55.1; H, 5.0; N, 13.0; Cl, 15.9; S, 7.4; H₂O, 4.3%
   Calculated for C₂₀H₁₈N₄S.2HCl.H₂O: C, 54.9; H, 5.1; N, 12.8; Cl, 16.2; S, 7.3; H₂O, 4.1%].
iii) By proceeding in the same manner as part (i), but replacing the 1-methyl-2-chloromethylimidazole hydrochloride by 1-benzyl-2-chloromethylimidazole hydrochloride, there was prepared 2-[(1-benzylimidazol-2-yl)methylthio]-4,5-diphenylimidazole, m.p. 181-183°C;
   [Found:- C, 73.8; H, 5.2; N, 13.3; S, 7.4%
   Calculated for C₂₆H₂₂N₄S:- C, 73.9; H, 5.3; N, 13.3; S, 7.6%
   N.M.R. (200MHz; CDCl₃): 4.20 (2H, s), 5.20 (2H, s), 6.92 (1H, d, J=2Hz), 7.07 (1H, d, J=2Hz), 7.10-7.60 (15H, m)
   IR (KBr): 687, 1110, 1490 cm⁻¹].
iv) By proceeding in the same manner as part (i), but replacing the 1-methyl-2-chloromethylimidazole hydrochloride by 4-chloromethylpyridine hydrochloride and carrying out the reaction in the presence of triethylamine, there was prepared 2-[pyrid-4-ylmethylthio]-4,5-diphenylimidazole.

This was converted to the hydrochloride salt as in Example 1(i) to give 2-[pyrid-4-ylmethylthio]-4,5-diphenylimidazole dihydrochloride, m.p. 225-227°C;
[Found:- C, 60.3; H, 4.56; N, 10.1; S, 7.6; Cl, 17.0%
Calculated for C₂₁H₁₇N₃S.2HCl:- C, 60.57; H, 4.59; N, 10.09; S, 7.7; Cl, 17.0%].

### EXAMPLE 4

### Compounds 1Q to 1T

i) A solution of tetrazole (5.8g) in anhydrous dimethylformamide (75ml) was cooled in an ice bath and carefully treated with sodium hydride (80% dispersion in oil, 2.5g). After stirring for 30min, 1-bromo-3-chloropropane (13.0g) was added and the mixture stirred at room temperature overnight. Evaporation of the mixture gave a colourless semisolid which was partitioned between water (100ml) and ethyl acetate (100ml). The layers were separated and the organic layer washed with water (50ml), dried (MgSO₄) and evaporated to give a mixture of 1-(3-chloropropyl)tetrazole and 2-(3-chloropropyl)tetrazole (11.5g).
   Potassium t-butoxide (2.24g) was added to a stirred solution of 4,5-diphenylimidazole-2-thiol (5.04g) in anhydrous dimethylformamide (75ml). After stirring at room temperature for 10min, the mixture of 1-(3-chloropropyl)tetrazole and 2-(3-chloropropyl)tetrazole (3.3g) in dimethylformamide (5ml) was added and the mixture stirred at room temperature overnight. The mixture was evaporated to dryness and the residue partitioned between ethyl acetate (150ml) and water (75ml). The layers were separated and the organic layer was washed with water (75ml), dried (MgSO₄) and evaporated to give a yellow solid. TLC analysis (silica gel plates, ethyl acetate eluent) showed two components, rf0.4 and 0.7. The mixture was purified by flash chromatography (ethyl acetate) to give two fractions.
   The faster running fraction was purified by flash chromatography (4:1 dichloromethane / ethyl acetate as eluent) to give 2-[3-(tetrazol-1-yl)propylthio]-4,5-diphenylimidazole (1.8g), as a white solid, m.p. 98-99°C; [Found:- C,63.2;H,5.00;N,23.0;S,8.8%; Calculated for C₁₉H₁₈N₆S:- C,62.95;H,5.01;N,23.19; S,8.85%].
   The slower running fraction was recrystallised from ethyl acetate to give 2-[3-(tetrazol-2-yl)propylthio]-4,5-diphenylimidazole (1.8g), as a white crystalline solid, m.p. 162-163°C; [Found:- C,62.7; H,4.9;N,23.3;S,8.9%; Calculated for C₁₉H₁₈N₆S:-C,62.95;H,5.01;N,23.19;S,8.85%].
ii) By proceeding in a similar manner, but replacing the 1-bromo-3-chloropropane by 1-chloro-4-bromobutane, there were prepared:-
   2-[4-(tetrazol-1-yl)butylthio]-4,5-diphenylimidazole, m.p. 125-126°C; and
   2-[4-(tetrazol-2-yl)butylthio]-4,5-diphenylimidazole, m.p. 133-134°C.

### EXAMPLE 5

### Compounds 2A to 2I

A stirred solution of 3,5-dimethylpyrazole (9.6g) in anhydrous dimethylformamide (100ml), cooled in an ice-bath, was treated with potassium tert-butoxide (11.2g). The ice-bath was removed and the mixture was stirred for one hour, the temperature rising to room temperature. The mixture was then treated with 1-bromo-5-chloropentane (18.6g), and the mixture was stirred at room temperature overnight, to give "reaction mixture 1".

A mixture of 4,5-diphenylimidazole-2-thiol (25.2g) and potassium tert-butoxide (11.2g) in anhydrous dimethylfornamide (200ml) was stirred at room temperature for 30 minutes. This mixture was treated with "reaction mixture 1", and stirred at room temperature overnight. The mixture was filtered and the filtrate was evaporated to low bulk, and treated with dichloromethane (400ml) and water (200ml). The organic layer was washed with water (200ml), dried over magnesium sulphate, and evaporated. The resulting residue was triturated with diethyl ether (250ml), and filtered. The filtrate was evaporated and the resulting residue was subjected to flash chromatography on a silica gel column, using a mixture of ethyl acetate and dichloromethane (1:1v/v) as eluent. The resulting product was triturated with pentane, to give 2-[5-(3,5-dimethylpyrazol-1-yl)pentylthio]-4,5-diphenylimidazole (16.9g), in the form of a white solid, m.p. 75°C. [Elemental analysis:-C,71.9;H,6.78;N,13.5;S,8.0%; calculated:- C,72.1; H,6.73;N,13.5;S,7.69%].

By proceeding in a similar manner, but replacing the 3,5-dimethylpyrazole, used as a starting material, by the corresponding quantity of the appropriate imidazole or the appropriate pyrazole, or derivative thereof, and replacing where necessary the 1-bromo-5-chloropentane, used as a starting material, by the corresponding quantity of the appropriate dihaloalkane, there were prepared:-
2-[5-(2-ethylimidazol-1-yl)pentylthio]-4,5-diphenylimidazole, m.p. 90°C;
2-[5-(imidazol-1-yl)pentylthio]-4,5-diphenylimidazole, m.p. 120°C;
2-[5-(2-methylimidazol-1-yl)pentylthio]-4,5-diphenylimidazole, m.p. 120°C;
2-[6-(2-methylimidazol-1-yl)hexylthio]-4,5-diphenylimidazole, m.p. 110°C;
2-[6-(2-ethylimidazol-1-yl)hexylthio]-4,5-diphenylimidazole, m.p. 105°C;
2-[5-(pyrazol-1-yl)pentylthio]-4,5-diphenylimidazole, m.p. 120°C;
2-[6-(imidazol-1-yl)hexylthio]-4,5-diphenylimidazole, m.p. 110°C; and
2-[6-(pyrazol-1-yl)hexylthio]-4,5-diphenylimidazole, m.p. 135°C.

### EXAMPLE 6

### Compounds 2J to 2T

A stirred solution of 3-methylpyrazole (10g) in anhydrous tetrahydrofuran (300ml) under nitrogen was carefully treated with sodium hydride (60% dispersion in oil, 6.7g). After stirring for 30 minutes at room temperature the mixture was treated with 1-bromo-5-chloropentane (23.4g), and the mixture was heated at reflux overnight, and then it was allowed to cool to room temperature. The resulting mixture was added to a stirred suspension of the sodium salt of 4,5-diphenylimidazole-2-thiol in anhydrous tetrahydrofuran [prepared by carefully treating a suspension of 4,5-diphenylimidazole-2-thiol (30.7g) in anhydrous tetrahydrofuran (300ml), under nitrogen, with sodium hydride (60% dispersion in oil, 5.7g) and stirring at room temperature for one hour] and the whole was heated at reflux overnight. Evaporation, followed by treatment with a mixture of ethyl acetate and water (500ml;1:1v/v), separation of the organic phase and subsequent concentration, gave a brown oil. This brown oil was treated with aqueous hydrochloric acid (800ml;2N) and charcoal (2g) at 90°C and the mixture was filtered. The filtrate was basified by addition of solid potassium carbonate, and was extracted four times with dichloromethane (300ml). The combined dichloromethane extracts were dried over magnesium sulphate and evaporated, and the residue was purified by flash chromatography on silica gel (using a mixture of ethyl acetate and cyclohexane, 1:2v/v as eluent). The major component was triturated with pentane and then with diethyl ether, to give a mixture of 2-[5-(3-methylpyrazol-1-yl)pentylthio]-4,5-diphenylimidazole and 2-[5-(5-methylpyrazol-1-yl)pentylthio]-4,5-diphenylimidazole (14.5g; ratio approximately 3:5) in the form of a white solid, m.p. 114-116°C; [Elemental analysis:- C, 71.6; H, 6.47; N, 14.1; S, 8.0%; Calculated:- C, 71.6; H, 6.5; N, 13.9; S, 7.96%].

By proceeding in a similar manner, but replacing the 3-methylpyrazole, used as a starting material, by the corresponding quantities of the appropriate azoles, there were prepared:-
2-[5-(3-amino-5-methylpyrazol-1-yl)pentylthio]-4,5-diphenylimidazole, m.p.60-62°C, [Elemental analysis:-C, 68.7; H, 6.46; N, 16.6%; Calculated:- C, 69.0; H, 6.51; N, 16.77%];
2-[5-(4-bromo-3,5-dimethylpyrazol-1-yl)pentylthio]-4,5-diphenylimidazole, m.p.122-124°C; [Elemental analysis:- C, 60.7; H, 5.46; N, 11.2; S, 6.42%; Calculated:- C, 60.6; H, 5.5; N, 11.3; S, 6.4%]; 2-[5-(3,5-diphenylpyrazol-1-yl)pentylthio]-4,5-diphenylimidazole, m.p.148-150°C;
2-[5-(pyrrol-1-yl)pentylthio]-4,5-diphenylimidazole, m.p. 132-134°C; [Elemental analysis:- C, 74.6; H, 6.54; N, 11.0; S, 8.3%; Calculated:- C, 74.37; H, 6.5; N, 10.84; S, 8.27%];
2-[5-(2,5-dimethylpyrrol-1-yl)pentylthio]-4,5-diphenylimidazole, m.p. 122-124°C; [Elemental analysis:- C, 74.8; H, 6.97; N, 9.9; S, 8.0%; Calculated:- C, 75.14; H, 7.03; N, 10.11; S, 7.71%];
By again proceeding in a similar manner, but replacing the 3-methylpyrazole by 3,5-dimethylpyrazole, and replacing the 1-bromo-5-chloropentane by the appropriate dihalide, there were prepared:-2-[4-(3,5-dimethylpyrazol-1-yl)butylthio]-4,5-diphenylimidazole, m.p.172-175°C; [Elemental analysis:- C, 71.6; H, 6.59; N, 13.9; S, 7.9%; Calculated:- C, 71.6; H, 6.5; N, 13.9; S, 7.96%];
2-[6-(3,5-dimethylpyrazol-1-yl)hexylthio]-4,5-diphenylimidazole, as a colourless viscous liquid.

By again proceeding in a similar manner, but replacing the 3-methylpyrazole by 3,5-dimethylpyrazole and replacing the 4,5-diphenylimidazole-2-thiol by the appropriate 4,5-diphenylimidazole-2-thiol, there were prepared:-
2-[5-(3,5-dimethylpyrazol-1-yl)pentylthio]-4,5-(bis-4-fluorophenyl)imidazole, as a viscous gum;
2-[5-(3,5-dimethylpyrazol-1-yl)pentylthio]-4,5-(bis-4-methylphenyl)imidazole, as a viscous gum; and
2-[5-(3,5-dimethylpyrazol-1-yl)pentylthio]-4,5-(bis-4-methoxyphenyl)imidazole, m.p.133-136°C.

### EXAMPLE 7

### Compounds 2RA and 2SA

2-[5-(3,5-Dimethylpyrazol-1-yl)pentylthio]-4,5-(bis-4-fluorophenyl)imidazole was dissolved in anhydrous tetrahydrofuran and treated with excess ethereal hydrogen chloride. Evaporation of this solution, trituration of the residue with toluene and then with diethyl ether, followed by crystallisation from a mixture of ethanol, acetone and diethyl ether, gave 2-[5-(3,5-dimethylpyrazol-1-yl)pentylthio]-4,5-(bis-4-fluorophenyl)imidazole hydrochloride as a white solid, m.p.159-162°C; [Elemental analysis:- C, 57.0; H, 5.5; N, 10.8; Cl, 13.6; S, 6.56%; Calculated for C₂₅H₂₆F₂N₄S.2HCl:- C, 57.1; H,5.37; N, 10.7; Cl, 13.5; S, 6.1%].

By proceeding in a similar manner, but crystallising from a mixture of ethanol and diethyl ether, 2-[5-(3,5-dimethylpyrazol-1-yl)pentylthio]-4,5-(bis-4-methylphenyl)imidazole was converted to 2-[5-(3,5-dimethylpyrazol-1-yl)pentylthio]-4,5-(bis-4-methylphenyl)imidazole hydrochloride as a white solid, m.p. 144-149°C.

### EXAMPLE 8

### Compound 2AA

2-[5-(3,5-Dimethylpyrazol-1-yl)pentylthio]-4,5-diphenylimidazole was dissolved in anhydrous tetrahydrofuran and treated with excess ethereal hydrogen chloride. Evaporation of this solution, followed by trituration of the residue with diethyl ether, gave 2-[5-(3,5-dimethylpyrazol-1-yl)pentylthio]-4,5-diphenylimidazole dihydrochloride, m.p.198-200°C.

### EXAMPLE 9

### Compound 2U

A mixture of 2-chloromethylimidazole hydrochloride (1.9g) and 4,5-diphenylimidazole-2-thiol (3.48g) in dimethylformamide (50ml) was heated at 100-110°C for 90 minutes and was then it was poured into water (300ml). The solid which precipitated was removed by filtration and the filtrate was treated with sodium hydrogen carbonate (3.1g), to give a precipitate. This precipitate was washed with water, and then recrystallised from ethanol, to give 2-(4,5-diphenylimidazol-2-ylthiomethyl]imidazole (2.5g), in the form of a white solid, m.p. 220-222°C.

### EXAMPLE 10

### Compound 2UA

A solution of maleic acid (1.68g) in ethyl acetate (50ml) was added to a boiling suspension of 2-(4,5-diphenylimidazol-2-ylthiomethyl)imidazole (2.45g) in ethyl acetate (200ml) and butan-2-one (100ml), producing a clear solution. The solution was concentrated under reduced pressure to about 50ml, returned to the boil, and treated with acetone, to give a clear solution, which was allowed to cool, to give 2-(4,5-diphenylimidazol-2-ylthiomethyl)imidazole dimaleate, in the form of white crystals, m.p. 160-162°C (with decomposition).

### EXAMPLE 11

### Compound 2V

2-(3-Hydroxypropyl)-1-methylimidazole (2.6g) was carefully treated with thionyl chloride (10ml), cooling in an ice-bath, and the resulting solution was left at room temperature for 3 days. Excess thionyl chloride was then removed under reduced pressure, and the residue was treated with dry toluene, to give a sticky solid, which was dried at 60°C/20mmHg. It was then dissolved in dry dimethylformamide (80ml) and treated with 4,5-diphenylimidazole-2-thiol (2.68g), and the mixture was heated at 120-140°C for 4 hours. The mixture was then cooled to 100°C and was then poured into a mixture of water (300ml) and hydrochloric acid (50ml;1N). The solid which precipitated was washed with water, and then it was discarded. The combined filtrate was basified to pH8 and the resulting precipitate was collected, washed with water, and crystallised from acetone, to give 4,5-diphenyl-2-[3-(1-methylimidazol-2-yl)propylthio]imidazole (1.81g), in the form of colourless needles, m.p. 174-176°C.

### EXAMPLE 12

### Compound 2W

A suspension of 2-[(5-(3,5-dimethylpyrazol-1-yl)pent-1-yl)thio]-4,5-diphenylimidazole (5.0g) in a mixture of dichloromethane (75ml) and water (75ml) was stirred at room temperature for 30 minutes. The mixture was then cooled to 5°C treated with a solution of sodium metaperiodate (10.9g) in water (25ml), followed immediately by ruthenium (III) chloride trihydrate (0.03g). The mixture was stirred at 5°C for 30 minutes and then it was treated carefully with solid sodium hydrogen carbonate (5.0g) and dichloromethane (100ml). The aqueous layer was extracted with dichloromethane (2x50ml). The combined extracts were layers were washed with water (2x50ml), dried over magnesium sulphate and evaporated, to give a dark oil. This oil was subjected to flash chromatography on silica gel, eluting with a mixture of ethyl acetate and cyclohexane (1:1v/v), followed by trituration with diethyl ether, to give 2-[(5-(3,5-dimethylpyrazol-1-yl)pent-1-yl)sulphonyl]-4,5-diphenylimidazole (1.5g) as a white powder, m.p. 142-144°C. [Elemental analysis:-Found:- C,67.2;H,6.35;N,12.5;S,7.2%; calculated:-C,66.93;H,6.29;N,12.49;S,7.14%;].

### EXAMPLE 13

A stirred solution of 2-[(5-(3,5-dimethylpyrazol-1-yl)pent-1-yl)thio]-4,5-diphenylimidazole (5.0g) in dichloromethane (250ml), cooled with in an acetone/ice bath (internal temperature -10 to -5°C) was treated with sodium hydrogen carbonate (2.0g), followed by 3-chloroperoxybenzoic acid (50-60%; 3.4g). After 35 minutes stirring in the cooling bath, the mixture was treated with sodium bicarbonate (5.0g) and dichloromethane (200ml). The organic layer was washed successively with saturated aqueous sodium metabisulphite solution (4x100ml), saturated aqueous sodium bicarbonate solution (100ml) and water (2x100ml), dried over magnesium sulphate and evaporated, to give a dark oil, which was subjected to flash chromatography on silica gel, eluting with a mixture of ethyl acetate and methanol (9:1v/v), to give 2-[(5-(3,5-dimethylpyrazol-1-yl)pent-1-yl)sulphinyl]-4,5-diphenylimidazole (1.2g) as an amorphous solid, m.p. 38-40°C.

The present invention also includes within its scope pharmaceutical formulations which comprise at least one of the compounds of formula (I) or a pharmaceutically acceptable salt thereof in association with a pharmaceutically acceptable carrier or coating. In clinical practice the compounds of the present invention may be administered parenterally, rectally or orally.

Solid compositions for oral administration include compressed tablets, pills, powders and granules. In such solid compositions, one or more of the active compounds is, or are, admixed with at least one inert diluent such as starch, sucrose or lactose. The compositions may also comprise, as is normal practice, additional substances other than inert diluents, e.g. lubricating agents, such as magnesium stearate.

Liquid compositions for oral administration include pharmaceutically acceptable emulsions, solutions, suspensions, syrups and elixirs containing inert diluents commonly used in the art such as water and liquid paraffin. Besides inert diluents such compositions may comprise adjuvants, such as wetting and suspending agents, and sweetening, flavouring, perfuming and preserving agents. The compositions according to the invention for oral administration also include capsules of absorbable material such as gelatin, containing one or more of the active substances with or without the addition of diluents or excipients.

Compositions according to the invention for parenteral administration include sterile aqueous, aqueous-organic, and organic solutions, suspensions and emulsions. Examples of organic solvents or suspending media are propylene glycol, polyethylene glycol, vegetable oils such as olive oil and injectable organic esters such as ethyl oleate. The compositions may also contain adjuvants such as stabilising, preserving, wetting, emulsifying and dispersing agents. They may be sterilised, for example, by filtration through a bacteria-retaining filter, by incorporation in the compositions of sterilising agents, by irradiation or by heating. They may also be manufactured in the form of sterile solid compositions, which can be dissolved in sterile water or some other sterile injectable medium immediately before use.

Solid compositions for rectal administration include suppositories formulated in accordance with known methods and containing at least one compound of formula (I) or a pharmaceutically acceptable salt thereof.

The percentage of active ingredient in the compositions of the invention may be varied, it being necessary that it should constitute a proportion such that a suitable dosage shall be obtained. Obviously, several unit dosage forms may be administered at about the same time. The dose employed will be determined by the physician, and depends upon the desired therapeutic effect, the route of administration, the duration of the treatment and the condition of the patient. In the adult, the doses are generally from 0.01 to 100, preferably 0.1 to 70, more especially 0.5 to 10, mg/kg body weight per day by oral administration, and from 0.001 to 10, preferably 0.01 to 1, mg/kg body weight per day by intravenous administration.

The following Example illustrates a pharmaceutical composition according to the present invention.

### COMPOSITION EXAMPLE

No. 2 size gelatin capsules each containing:-

| | |
|---|---|
| 2-[5-(1,2,4-triazol-1-yl)pentylthio]-4,5-diphenylimidazole dihydrochloride | 20 mg |
| lactose | 100 mg |
| starch | 60 mg |
| dextrin | 40 mg |
| magnesium stearate | 1 mg |

were prepared in accordance with the usual procedure.

## Claims

1. Use of an imidazole derivative of the general formula :
[DPIM]-S(O)ₚ-W-Y I
wherein [DPIM] is of the general formula : 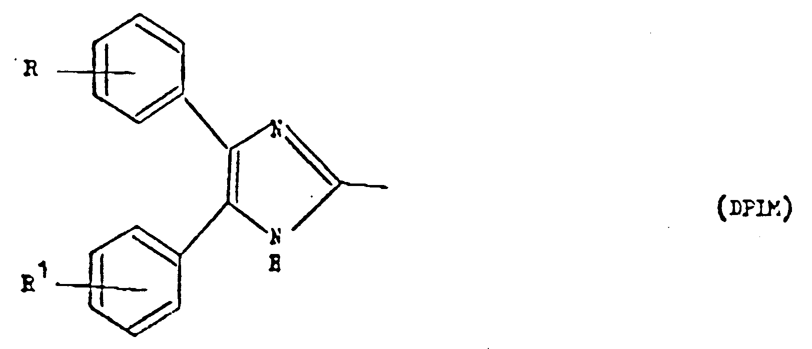 wherein R and R¹ are the same or different and each represents a hydrogen or halogen atom or a straight- or branched-chain alkyl or alkoxy group containing from 1 to 6 carbon atoms, p represents 0, 1 or 2, W represents a methylene group or an alkylene chain of 2 to 6 carbon atoms optionally substituted with one or more straight- or branched-chain alkyl groups of 1 to 4 carbon atoms, Y represents a 5- or 6-membered unsaturated ring containing 1, 2, 3 or 4 nitrogen atoms, optionally substituted by one or more substituents selected from straight- and branched-chain alkyl groups containing from 1 to 6 carbon atoms (which are each optionally substituted by a phenyl group), phenyl groups, amino groups and nitro groups, and pharmaceutically acceptable acid addition salts thereof, provided that when p represents 0 or 1 and W represents an unsubstituted methylene group, Y represents other than a 2-pyridyl group for the preparation of a pharmaceutical composition for the treatment of a condition which can be ameliorated by administration of an inhibitor of acyl coenzyme-A:cholesterol-O-acyl transferase or of an inhibitor of the binding of thromboxane TxA₂ to its receptors.

2. Use according to claim 1 for use in the preparation of a pharmaceutical composition for the treatment of atherosclerosis, hyperlipidaemia, cholesterol ester storage disease, atheroma in vein grafts, thrombosis, myocardial infarction, a vasospastic disorder, bronchospasm, or in reperfusion salvage therapy.

3. An imidazole derivative of general formula I, as defined in claim 1, or a pharmaceutically acceptable acid addition salt thereof, subject to the provisos that (i) when p represents 0 or 1 and W represents an unsubstituted methylene group or an unsubstituted alkylene chain of 2 to 4 carbon atoms, Y represents other than a pyridyl, pyrimidinyl or imidaxolinyl group, substituted or unsubstituted with one or two alkyl groups, R and R¹ being as defined in claim 1, and (ii) when R and R¹ each represent hydrogen atoms, p represents 0 or 1 and W represents an unsubstituted methylene group, Y represents other than a pyrid-2-yl group, substituted or unsubstituted with 1, 2 or 3 alkyl groups each containing up to 4 carbon atoms, for use in a method of treatment of the human or animal body by therapy.

4. A compound according to claim 3 for use in a method of treatment of the human or animal body by therapy of atherosclerosis, hyperlipidaemia, cholesterol ester storage disease, atheroma in vein grafts, thrombosis, myocardial infarction, a vasospastic disorder, bronchospasm, or in reperfusion salvage therapy.

5. An imidazole derivative of general formula I as defined in claim 1 or a pharmaceutically acceptable acid addition salt thereof, provided that : (i) when p represents 0 or 1 and W represents an unsubstituted methylene group or an unsubstituted alkylene chain of 2 to 4 carbon atoms, Y represents other than a pyridyl, pyrimidinyl or imidazolinyl group, substituted or unsubstituted with one or two alkyl groups, R and R¹ being as defined in claim 1, and (ii) when R and R¹ each represent hydrogen atoms, p represents 0 or 1 and W represents an unsubstituted methylene group, Y represents other than a pyrid-2-yl group, substituted or unsubstituted with 1, 2 or 3 alkyl groups each containing up to 4 carbon atoms.

6. A compound according to claim 5 wherein alkyl and alkoxy groups within the definitions of R and R¹ contain from 1 to 3 carbon atoms.

7. A compound according to claim 5 or 6 wherein at least one of the symbols has a value selected from the following :
(i) R represents a hydrogen or fluorine atom or a methyl or methoxy group;
(ii) R¹ represents a hydrogen or fluorine atom or a methyl or methoxy group;
(iii)W represents a methylene or ethylidene group or an alkylene chain of 2 to 6 carbon atoms;
(iv) Y represents an imidazolyl, pyrazolyl, triazolyl, tetrazolyl, pyridyl or pyrrolyl group, optionally substituted by one or two substituents selected from methyl, ethyl, benzyl, phenyl, amino and nitro groups.

8. A compound according to claim 7 wherein Y represents an imidazol-1-yl, imidazol-2-yl, pyrazol-1-yl, 1,2,4-triazol-1-yl, tetrazol-1-yl, tetrazol-2-yl, pyrid-4-yl, or pyrrol-1-yl group optionally substituted by one or two substituents from methyl, ethyl, benzyl, phenyl, amino and nitro groups.

9. A compound according to claim 5 hereinbefore identified as any one of compounds 1A to 2X.

10. A process for the preparation of a compound of general formula I as defined in claim 5 which comprises:
(A) the reaction of a compound of the general formula:
[DPIM]-SH II
or a salt thereof, wherein [DPIM] is as defined in claim 5, with a compound of the general formula :
X-W-Y III
or a salt thereof, wherein X is a group displaceable by a thiolate salt and W and Y are as defined in claim 5;
(B) when [DPIM], R, R¹, W and Y are as defined in claim 5 and p represents 1 or 2, the oxidation of a compound of general formula I wherein [DPIM], R, R¹, W and Y are as defined in claim 5 and p is less than in the desired product;
optionally followed by the conversion of a compound of general formula I into a pharmaceutically acceptable acid addition salt thereof.

11. A pharmaceutical composition which comprises an imidazole derivative of general formula I as defined in claim 5, or a pharmaceutically acceptable acid addition salt thereof, in association with a pharmaceutically acceptable carrier or coating.

## Patentansprüche

1. Verwendung eines Imidazolderivats der allgemeinen Formel:
[DPIM]-S(O)ₚ-W-Y (I)
worin [DPIM] der allgemeinen Formel: 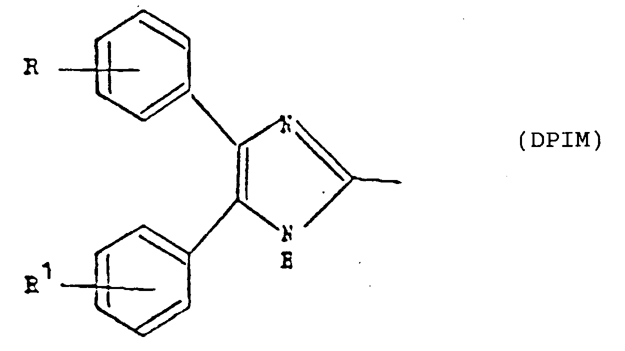 mit R und R¹, die gleich oder verschieden sind, jeweils gleich einem Wasserstoff- oder Halogenatom oder einer gerad- oder verzweigtkettigen Alkyl- oder Alkoxygruppe mit 1 bis 6 Kohlenstoffatom(en), entspricht, p = 0, 1 oder 2 ist, W eine Methylengruppe oder eine Alkylenkette von 2 bis 6 Kohlenstoffatomen, die gegebenenfalls durch eine oder mehrere gerad- oder versweigtkettige Alkylgruppe(en) mit 1 bis 4 Kohlenstoffatom(en) substituiert ist, bedeutet und Y einen 5- oder 6-gliedrigen ungesättigten Ring mit 1, 2, 3 oder 4 Stickstoffatom(en), der gegebenenfalls durch einen oder mehrere Substituenten, ausgewählt aus gerad- und verzweigtkettigen Alkylgruppen mit 1 bis 6 Kohlenstoffatom(en), (die ihrerseits jeweils durch eine Phenylgruppe substituiert sein können), Phenylgruppen, Aminogruppen und Nitrogruppen, substituiert ist, darstellt, wobei gilt, daß im Falle, daß p = 0 oder 1 ist und W für eine unsubstituierte Methylengruppe steht, Y eine von einer 2-Pyridylgruppe verschiedene Gruppe darstellt,
und pharmazeutisch akzeptabler Säureadditionssalze desselben
zur Herstellung einer Arzneimittelzubereitung zur Behandlung einer Störung, die durch Verabreichung eines Hemmstoffs oder Inhibitors für Acylcoenzym-A:Cholesterin-O-acyltransferase oder eines Hemmstoffs oder Inhibitors für die Bindung von Thromboxan TxA₂ an seine Rezeptoren gelindert bzw. gebessert werden kann.

2. Verwendung einer Verbindung nach Anspruch 1 zur Herstellung einer Arzneimittelzubereitung zur Behandlung von Atherosklerose, Hyperlipidämie, krankhafter Cholesterinesterablagerungen, Atherom bei Gefäßplastiken, Thrombose, Herzinfarkt, Gefäßkrämpfen, Bronchialkrämpfen oder bei der Reperfusionsrettungstherapie.

3. Imidazolderivat der allgemeinen Formel (I) entsprechend der Definition in Anspruch 1 oder eines pharmazeutisch akzeptablen Säureadditionssalzes desselben mit folgenden Ausnahmeregelungen:
(i) wenn p = 0 oder 1 ist und W für eine unsubstituierte Methylengruppe oder eine unsubstituierte Alkylenkette mit 2 bis 4 Kohlenstoffatomen steht, bedeutet Y eine von einer ein- oder zweifach alkylsubstituierten oder unsubstituierten Pyridyl-, Pyrimidinyl- oder Imidazolinylgruppe verschiedene Gruppe und entsprechen R und R¹ der Definition in Anspruch 1 und
(ii) wenn R und R¹ jeweils für ein Wasserstoffatom stehen, p = 0 oder 1 ist und W eine unsubstituierte Methylengruppe bedeutet, steht Y für eine von einer mit 1, 2 oder 3 Alkylgruppe(n) mit jeweils bis zu 4 Kohlenstoffatomen substituierten oder unsubstituierten Pyrid-2-yl-gruppe verschiedene Gruppe,
zur Verwendung im Rahmen eines Verfahrens zur therapeutischen Behandlung des menschlichen oder tierischen Körpers.

4. Verbindung nach Anspruch 3 zur Verwendung im Rahmen eines Verfahrens zur therapeutischen Behandlung des menschlichen oder tierischen Körpers, nämlich von Atherosklerose, Hyperlipidämie, krankhaften Cholesterinesterablagerungen, Atherom bei Gefäßplastiken, Thrombose, Herzinfarkt, Gefäßkrämpfen, Bronchialkrämpfen oder bei der Reperfusionsrettungstherapie.

5. Imidazolderivat der allgemeinen Formel (I) gemäß der Definition von Anspruch 1 oder pharmazeutisch akzeptables Säureadditionssalzs desselben, wobei folgende Ausnahmeregelungen gelten:
(i) wenn p = 0 oder 1 ist und W für eine unsubstituierte Methylengruppe oder eine unsubstituierte Alkylenkette mit 2 bis 4 Kohlenstoffatomen steht, bedeutet Y eine von einer ein- oder zweifach alkylsubstituierten oder unsubstituierten Pyridyl-, Pyrimidinyl- oder Imidazolinylgruppe verschiedene Gruppe und entsprechen R und R¹ der Definition in Anspruch 1 und
(ii) wenn R und R¹ jeweils für ein Wasserstoffatom stehen, p = 0 oder 1 ist und W eine unsubstituierte Methylengruppe bedeutet, steht Y für eine von einer mit 1, 2 oder 3 Alkylgruppe(n) mit jeweils bis zu 4 Kohlenstoffatomen substituierten oder unsubstituierten Pyrid-2-yl-gruppe verschiedene Gruppe.

6. Verbindung nach Anspruch 5, wobei die Alkyl- und Alkoxygruppen innerhalb der Definitionen für R und R¹ 1 bis 3 Kohlenstoffatom(en) aufweisen.

7. Verbindung nach Anspruch 5 oder 6, wobei mindestens eines der Symbole eine aus folgenden Bedeutungen ausgewählte Bedeutung besitzt:
(i) R steht für ein Wasserstoff- oder Fluoratom oder eine Methyl- oder Methoxygruppe;
(ii) R¹ steht für ein Wasserstoff- oder Fluoratom oder eine Methyl- oder Methoxygruppe;
(iii) W steht für eine Methylen- oder Ethylidengruppe oder eine Alkylenkette von 2 bis 6 Kohlenstoffatomen;
(iv) Y steht für eine gegebenenfalls durch einen oder zwei Substituenten, ausgewählt aus Methyl-, Ethyl-, Benzyl-, Phenyl-, Amino- und Nitrogruppen, substituierte Imidazolyl-, Pyrazolyl-, Triazolyl-, Tetrazolyl-, Pyridyl- oder Pyrrolylgruppe.

8. Verbindung nach Anspruch 7, wobei Y ein für eine gegebenenfalls durch einen oder zwei Substituenten, ausgewählt aus Methyl-, Ethyl-, Benzyl-, Phenyl-, Amino- und Nitrogruppen, substituierte Imidazol-1-yl-, Imidazol-2-yl-, Pyrazol-1-yl-, 1,2,4-Triazol-1-yl-, Tetrazol-1-yl-, Tetrazol-2-yl-, Pyrid-4-yl- oder Pyrrol-1-ylgruppe steht.

9. Verbindung nach Anspruch 5, nämlich eine der zuvor identifizierten Verbindungen 1A bis 2X.

10. Verfahren zur Herstellung einer Verbindung der allgemeinen Formel (I) entsprechend Anspruch 5 durch
(A) Umsetzung einer Verbindung der allgemeinen Formel:
[DPIM]-SH (II)
oder eines Salzes derselben, wobei [DPIM] gemäß Anspruch 5 definiert ist, mit einer Verbindung der allgemeinen Formel:
X-W-Y (III)
oder einem Salz derselben, worin X für eine durch ein Thiolatsalz ersetzbare Gruppe steht und W und Y die in Anspruch 5 angegebene Bedeutung besitzen;
(B) wenn [DPIM], R, R¹, W und Y die in Anspruch 5 angegebene Bedeutung besitzen und p = 1 oder 2 ist, Oxidation einer Verbindung der allgemeinen Formel (I), worin [DPIM], R, R¹, W und Y die in Anspruch 5 angegebene Bedeutung besitzen und p einen geringeren Wert als in dem gewünschten Produkt aufweist;
gegebenenfalls gefolgt von einer Umwandlung einer Verbindung der allgemeinen Formel (I) in ein pharmazeutisch akzeptables Säureadditionssalz derselben.

11. Arzneimittelzubereitung, umfassend ein Imidazolderivat der allgemeinen Formel (I) entsprechend der Definition in Anspruch 5 oder ein pharmazeutisch akzeptables Säureadditionssalz derselben in Verbindung mit einem pharmazeutisch akzeptablen Träger oder Uberzug.

## Revendications

1. Utilisation d'un dérivé d'imidazole de formule générale :
[DPIM]-S(O)ₚ-W-Y I
dans laquelle [DPIM] a pour formule générale : 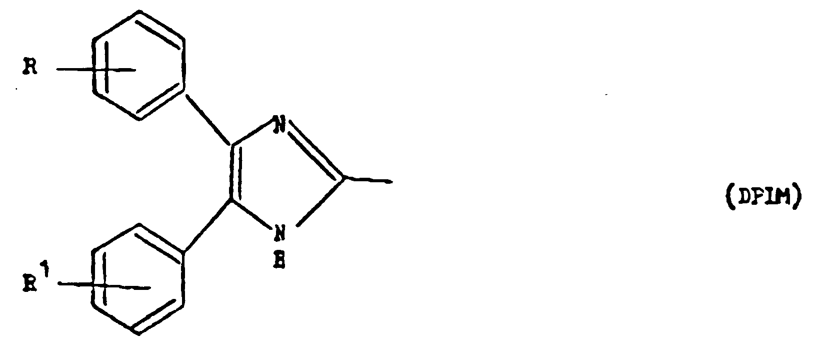 où R et R¹ sont identiques ou différents et représentent chacun un atome d'hydrogène ou d'halogène ou un groupe alkyle ou alcoxy linéaire ou ramifié contenant 1 à 6 atomes de carbone ; p vaut 0, 1 ou 2 ; W représente un groupe méthylène ou une chaîne alkylène de 2 à 6 atomes de carbone, éventuellement substituée par un ou plusieurs groupes alkyles linéaires ou ramifiés ayant 1 à 4 atomes de carbone ; Y représente un noyau insaturé, pentagonal ou hexagonal, contenant 1, 2, 3 ou 4 atomes d'azote, éventuellement substitué par un ou plusieurs substituants choisis parmi des groupes alkyles linéaires ou ramifiés contenant 1 à 6 atomes de carbone (qui sont chacun éventuellement substitués par un groupe phényle), des groupes phényles, des groupes amino et des groupes nitro, et ces sels d'addition d'acide pharmaceutiquement acceptables, à la condition que, lorsque p vaut 0 ou 1 et que W représente un groupe méthylène non substitué, Y représente un groupe 2-pyridyle, pour la préparation d'une composition pharmaceutique destinée à traiter un état qui peut être amélioré par administration d'un inhibiteur d'acyle-coenzyme-A : cholestérol-O-acyle transférase ou d'un inhibiteur de la fixation de thromboxane TxA₂ sur ses récepteurs.

2. Utilisation selon la revendication 1, destinée à servir à la préparation d'une composition pharmaceutique pour le traitement de l'athérosclérose, d'une hyperlipidémie, d'une maladie de mise en réserve ou stockage d'ester de cholestérol, d'athérome dans des greffes ou transplantations de veines, de thrombose, d'infarctus du myocarde, d'un trouble vasospastique, d'un spasme bronchique ou d'une thérapie de sauvetage avec reperfusion.

3. Dérivé d'imidazole de formule générale I, selon la définition donnée à la revendication 1, ou son sel d'addition d'acide pharmaceutiquement acceptable, à la condition que (i) quand p vaut 0 ou 1 et W représente un groupe méthylène non substitué ou une chaîne alkylène non substituée de 2 à 4 atomes de carbone, Y représente autre chose qu'un groupe pyridyle, pyrimidinyle ou imidazolinyle, non substitué ou substitué par un ou deux groupes alkyles ; R et R¹ étant comme définis à la revendication 1, et (ii) quand R et R¹ représentent chacun des atomes d'hydrogène, que p vaut 0 ou 1 et que W représente un groupe méthylène non substitué, Y représente autre chose qu'un groupe pyrid-2-yle non substitué ou substitué par 1, 2 ou 3 groupes alkyles contenant chacun jusqu'à 4 atomes de carbone, pour servir dans une méthode de traitement par thérapie du corps humain ou animal.

4. Composition selon la revendication 3, destinée à servir dans une méthode de traitement du corps humain ou animal, par thérapie de l'athérosclérose, d'une hyperlipidémie, d'une maladie de mise en réserve ou de stockage d'un ester de cholestérol, de l'athérome dans des greffes de veines, d'une thrombose, d'un infarctus du myocarde, d'un trouble vasospastique, d'un spasme bronchique ou d'une thérapie de sauvetage en cas de reperfusion.

5. Dérivé d'imidazole de formule générale I, selon la définition donnée à la revendication 1, ou un sel d'addition d'acide pharmaceutiquement acceptable de ce dérivé, à la condition que :
(i) quand p vaut 0 ou 1 et que W représente un groupe méthylène non substitué ou une chaîne alkylène non substituée de 2 à 4 atomes de carbone, Y représente autre chose qu'un groupe pyridyle, pyrimidinyle ou imidazolinyle, non substitué ou substitué par un ou deux groupes alkyles, R et R¹ étant comme défini à la revendication 1, et (ii) quand R et R¹ représente chacun des atomes d'hydrogène, que p vaut 0 ou 1 et que W représente un groupe méthylène non substituté, Y représente autre chose qu'un groupe pyrid-2-yle, substitué ou non substitué par 1, 2 ou 3 groupes alkyles contenant chacun jusqu'à 4 atomes de carbone.

6. Composé selon la revendication 5, dans lequel les groupes alkyles et alcoxy entrant dans la définition de R et R¹ contiennent de 1 à 3 atomes de carbone.

7. Composé selon la revendication 5 ou 6, dans lequel au moins l'un des symboles a une valeur choisie parmi les suivantes :
(i) R représente un atome d'hydrogène ou de fluor ou un groupe méthyle ou méthoxy ;
(ii) R¹ représente un atome d'hydrogène ou de fluor ou un groupe méthyle ou méthoxy ;
(iii) W représente un groupe méthylène ou éthylidène ou une chaîne alkylène ayant 2 à 6 atomes de carbone ;
(iv) Y représente un groupe imidazolyle, pyrazolyle, triazolyle, tétrazolyle, pyridyle ou pyrrolyle, éventuellement substitué par 1 ou 2 substituants choisis parmi des groupes méthyle, éthyle, benzyle, phényle, amino et nitro.

8. Composé selon la revendication 7, dans lequel Y représente un groupe imidazol-1-yle, imidazol-2-yle, pyrazol-1-yle, 1,2,4-triazol-1-yle, tétrazol-1-yle, tétrazol-2-yle, pyrid-4-yle, ou pyrrol-1-yle éventuellement substitué par 1 ou 2 substituants choisis parmi un groupe méthyle, éthyle, benzyle, phényle, amino et nitro.

9. Composé selon la revendication 5, identifié ci-dessus comme étant l'un quelconque des composés 1A à 2X.

10. Procédé de préparation d'un composé de formule générale I, selon la définition donnée à la revendication 5, qui comprend :
(A) la réaction d'un composé de formule générale :
[DPIM]-SH II
ou d'un de ses sels, formule dans laquelle [DPIM] est tel que défini dans la revendication 5, avec un composé de formule générale :
X-W-Y III
ou un de ses sels, formule dans laquelle X représente un groupe déplaçable par un thiolate salin, et W et Y sont comme définis à la revendication 5 ;
(B) quand [DPIM], R, R¹, W et Y sont tels que définis à la revendication 5 et que p représente 1 ou 2, l'oxydation d'un composé de formule générale I dans laquelle [DPIM], R, R¹, W et Y sont comme définis à la revendication 5 et p a une valeur inférieure à celle qu'il a dans le produit voulu ;
ce qui est éventuellement suivi par la conversion d'un composé de formule générale I quand un sel d'addition d'acide pharmaceutiquement acceptable de ce composé.

11. Composition pharmaceutique, qui comprend un dérivé d'imidazole de formule générale I, comme défini à la revendication 5, ou un sel d'addition d'acide pharmaceutiquement acceptable de ce dérivé, en association avec un excipient pour revêtement d'enrobage, pharmaceutiquement acceptables.
